# EUROPEAN PATENT APPLICATION

(11) **EP 4 704 095 A1**
(43) Date of publication of application: **04.03.2026**
(21) Application number: 25199399.4
(22) Date of filing: 01.09.2025
(51) Int. Cl.: G16C 20/20, G16C 20/70

(54) **RESIDUAL LOOP SPECTRAL SEARCH AND ANNOTATION FOR FRAGMENTATION IONS**

(30) Priority: 30.08.2024 US 202418821060
(71) Applicant: Thermo Fisher Scientific S.p.A, 20054 Segrate (MI) (IT); HighChem s.r.o., 821 09 Bratislava (SK)
(72) Inventor: ULASZEWSKA, Marynka, Milano (IT); RAAB, Michal, Bratislava (SK)
(74) Representative: Boult Wade Tennant LLP

(57) **Abstract**

Systems or techniques are provided for looping spectral searches of residual ions. In various embodiments, a scientific instrument can comprise a mass spectrometer. In various aspects, the scientific instrument can annotate one or more ion peaks of a fragmentation spectrum. In various aspects, the scientific instrument can remove the one or more annotated ion peaks from the fragmentation spectrum and resubmit the fragmentation spectrum for annotation, wherein one or more of the remaining ion peaks can be annotated.

## Description

### Background

Fragmentation spectra analysis search engines query unknown spectrums a single time to annotate ion peaks within the fragmentation spectrum. This can lead to cases where one or more ion peaks are left un-annotated, thereby limiting annotation coverage and decreasing the ability to annotate fragmentation spectra of complex molecules.

### Summary

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, devices, systems, computer-implemented methods, apparatus or computer program products that facilitate looped spectral searches for residual fragmentation ions are described.

According to one or more embodiments, a system is provided. The system can comprise a mass spectrometer. The scientific instrument can further comprise a non-transitory computer-readable memory that can store computer-executable components. The system can further comprise a processor that can be operably coupled to the non-transitory computer-readable memory and that can execute the computer-executable components stored in the non-transitory computer-readable memory. In various embodiments, the computer-executable components can comprise an annotation component that generates an annotated portion of the fragmentation spectrum based on previously removed annotated ion peaks of the fragmentation spectrum, wherein the annotated portion of the fragmentation spectrum comprises one or more ion peaks annotated as belonging to a first molecular structure. In various aspects, the computer-executable components can comprise a looping component that removes annotated ion peaks from the fragmentation spectrum and re-submits the fragmentation pattern to the annotation component for one or more additional iterations of annotation.

An advantage of the system, and/or of a corresponding computer-implemented method and/or computer program product can be the ability to repeatedly annotate ion peaks within a fragmentation spectrum, allowing for the identification of multiple molecules or structures within a fragmentation spectrum. This can allow for more accurate identification and analysis of material related to fragmentation and mass spectral analysis.

### Brief Description of the Drawings

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 is a block diagram of an example scientific instrument module for performing annotation operations, in accordance with various embodiments described herein.
FIG. 2 is a flow diagram of an example method of performing annotation operations, in accordance with various embodiments described herein.
FIGS. 3 and 4 illustrate block diagrams of example, non-limiting, scientific instruments that facilitate looped spectral searches of residual ions in accordance with one or more embodiments described herein.
FIG. 5 illustrates a flow diagram of a single spectral search in accordance with one or more embodiments described herein.
FIG. 6 illustrates a flow diagram of a spectral search with a single loop for a search of residual ions in accordance with one or more embodiments described herein.
FIGS. 7A and 7B illustrate a flow diagram of multiple search loops of residual ions in accordance with one or more embodiments described herein.
FIG. 8 illustrates a flow diagram of an example, non-limiting computer-implemented method that can facilitate looped annotation of fragmentation spectra of unknown samples in accordance with one or more embodiments described herein.
FIG. 9 illustrates a flow diagram of an example, non-limiting, computer-implemented method that can facilitate training of an annotation machine learning model in accordance with one or more embodiments described herein.
FIG. 10 illustrates an example, non-limiting, block diagram of a graphical user interface that can be used in the performance of some or all of the methods or techniques disclosed herein.
FIG. 11 illustrates an example, non-limiting, block diagram of a computing device that can perform some or all of the methods or techniques disclosed herein.
FIG. 12 illustrates an example, non-limiting, block diagram of a scientific instrument support system in which some or all of the methods or techniques disclosed herein may be performed.
FIG. 13 illustrates a block diagram of an example, non-limiting, operating environment in which one or more embodiments described herein can be facilitated.

### Detailed Description

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more embodiments are now described with reference to the drawings, wherein like reference numerals are utilized to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Within mass spectra analysis, samples can be analyzed by applying one or more fragmentation technologies and/or techniques to a sample. These fragmentation technologies cause chemical dissociation within the sample, thereby leading to ionization. A mass spectrometer can then measure or determine the fragmentation spectra through determining the relative ion abundance as a function of mass-to-charge ratio of the sample after fragmentation. The resulting fragmentation spectra comprises a graph of the mass to charge ratio of the fragmentation ions. The graph will exhibit various peaks (e.g., high abundances of various fragmentation ions) The fragmentation spectra can then be compared to fragmentation patterns (e.g., fragmentation spectra of known samples illustrating the relative abundances of fragmentation ions that the known sample tends to produce) of known molecules to generate annotated fragmentation spectra of the sample, enabling identification of the sample or a portion thereof by matching peaks of the fragmentation spectra of the unknown sample to peaks of fragmentation spectra of known samples.

Existing spectral library search engines perform a single query of a fragmentation spectrum of an unknown molecular sample, annotating ion peaks of the fragmentation spectrum based on a comparison to known fragmentation patterns. However, with some molecular samples, the search function may only annotate a portion of the fragmentation spectrum of a molecular sample based on a closest known fragmentation pattern, leaving a significant number of ion peaks of the fragmentation spectrum un-annotated. This produces results that may only identify some of the ion fragments of the sample, thereby limiting the usefulness of such analysis.

To overcome the one or more deficiencies of existing spectral library search engines as identified above, one or more embodiments described herein can annotate one or more ion peaks of a fragmentation spectrum of a molecular structure as belonging to a first molecular substructure, remove the one or more annotated ion peaks from the fragmentation spectrum, and annotate one or more remaining ion peaks in the fragmentation spectrum as belonging to a second molecular substructure, based on the removed annotated ion peaks. By removing the previously annotated ion peaks, the search function can find a closest known fragmentation pattern for the remaining ion peaks by querying only those remaining ion peaks, that the search engine may not have otherwise identified due to the presence of the previously annotated ion peaks.

Furthermore, a subsequent round of annotation can select a second molecular substructure that is likely to be present based on the presence of the first molecular substructure. In one or more embodiments, residual ion peaks can be queried in a looped fashion and this looping search can be repeated multiple times until one or more defined search criteria have been met. Examples of defined search criteria comprise, but are not limited to, a defined number of loop iterations, a defined amount of search time, until a defined percentage or number of ion peaks within the fragmentation spectrum are annotated, or another defined search criteria. As a result of this looping search of residual ion peaks, a higher percentage of the ion peaks within a fragmentation spectrum can be annotated leading to more robust analysis of the originally-presented fragmentation spectrum. For example, portions of more complex molecules often exhibit fragmentation spectra similar to fragmentation patterns of known smaller compounds, which may account for the various molecular substructures of the more complex molecules. Accordingly, the techniques described herein can enable annotation and/or identification of multiple molecular substructures of a complex molecule (e.g., greater annotation coverage), wherein existing techniques would only allow for annotation of a single molecular substructure. By enabling annotation/identification of multiple molecular substructures within complex molecules, a more accurate analysis and/or understanding of the complex molecule's composition and structure can be achieved.

One or more embodiments are now described with reference to the drawings, where like referenced numerals are used to refer to like elements throughout. In the following description, for purposes of explanation, numerous specific details are set forth to provide a more thorough understanding of the one or more embodiments. It is evident in various cases, however, that the one or more embodiments can be practiced without these specific details.

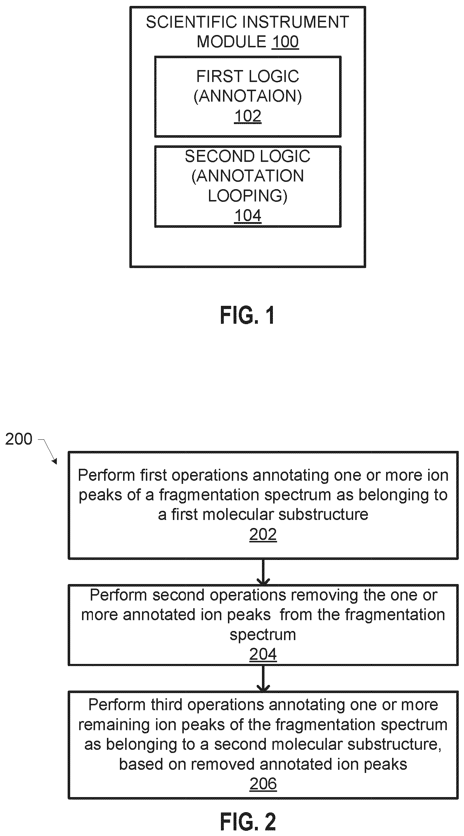

FIG. 1 illustrates an example, non-limiting block diagram of a scientific instrument module 100 in accordance with various embodiments described herein.

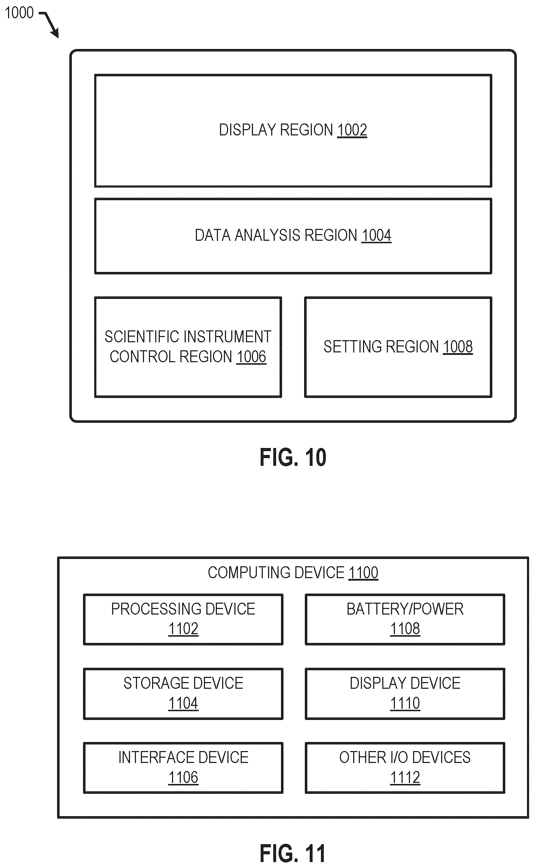

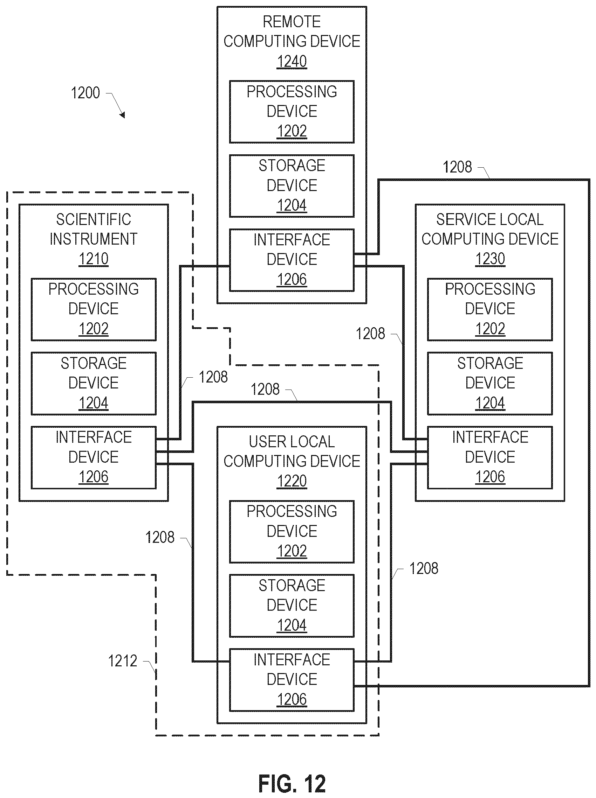

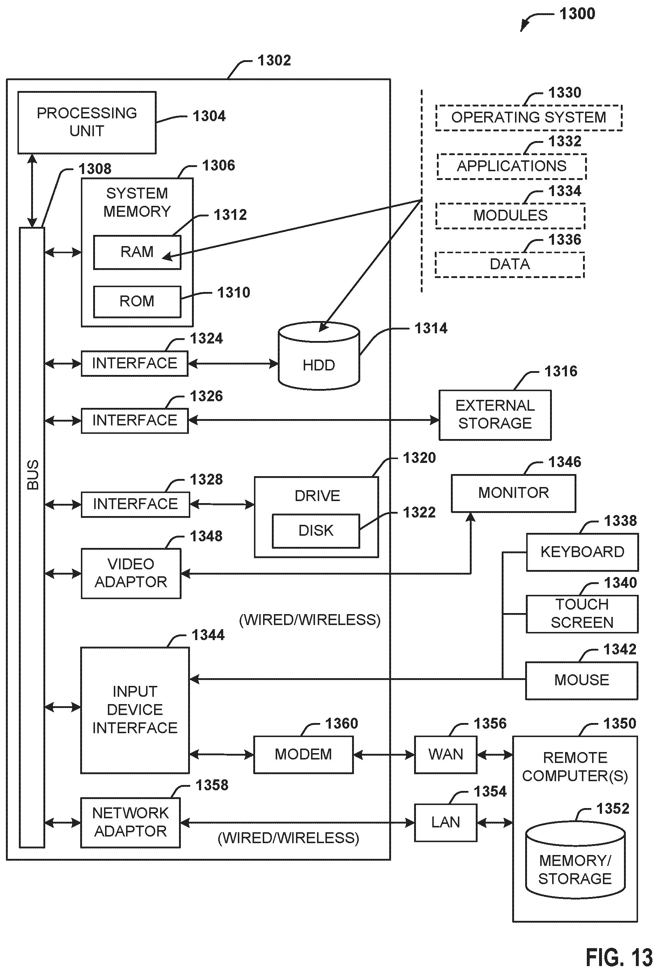

In various embodiments, the scientific instrument module 100 can be implemented by circuitry (e.g., including electrical or optical components), such as a programmed computing device. Logic of the scientific instrument module 100 can be included in a single computing device or can be distributed across multiple computing devices that are in communication with each other as appropriate. Examples of computing devices that may, singly or in combination, implement the scientific instrument module 100 are discussed herein with reference to FIGS. 11 and 13, and examples of systems or networks of interconnected computing devices, in which the scientific instrument module 100 may be implemented across one or more of the computing devices, are discussed herein with reference to FIG. 12.

The scientific instrument module 100 may include first logic 102 and second logic 104. As used herein, the term "logic" may include an apparatus that is to perform a set of operations associated with the logic elements. For example, any of the logic elements included in the scientific instrument module 100 may be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

In various embodiments, there can be a scientific instrument corresponding to the scientific instrument module 100. In various aspects, the scientific instrument can be any suitable computerized device that can electronically measure some scientifically-relevant, clinically-relevant, or research-relevant characteristic, property, or attribute of an analytical sample (e.g., of a known or unknown mixture, compound, or collection of matter). As a non-limiting example, a scientific instrument can be a mass spectrometer. In such a case, the scientific instrument can measure or determine ion spectra (e.g., relative ion abundance as a function of mass-to-charge ratio) of the analytical sample.

The first logic 102 may annotate one or more ion peaks of a fragmentation spectrum. As an example, a fragmentation spectrum of a sample can be obtained by applying one or more fragmentation techniques or technologies (e.g., Collision-induced dissociation, Higher-energy collisional dissociation, Ultraviolet photodissociation, electron transfer dissociation, electron capture dissociation or any other fragmentation technique or technology) to the sample and then using a mass analyzer technique to measure or determine relative ion abundance as a function of mass-to-charge ratio. The fragmentation spectrum can then be compared with known fragmentation patterns stored within a library using two steps: 1) selection of a comparison direction between the query and library (e.g., forward, reversed or symmetric) and the selection of a scoring algorithm that will compare ion intensities, mass accuracy and/or other factors between the fragmentation spectrum of the sample and ion intensities, mass accuracy and/or other factors of known fragmentation patterns within the library. Ion peaks are then matched with those from known fragmentation patterns of known samples or compounds to identify a molecular structure and the matched ion peaks are annotated with names based on the identified molecular structure, producing an annotated fragmentation spectrum. For example, the annotation can comprise comparing, by a device, one or more ion peaks of the fragmentation spectrum of the unknown sample to one or more ion peaks of one or more possible match fragmentation patterns stored in a fragmentation pattern library, wherein the pattern library stores fragmentation patterns of known samples or compounds, selecting, by the device and/or an entity, a match fragmentation pattern from the one or more possible match fragmentation patterns, and marking, by the device, the one or more matched ion peaks of the fragmentation spectrum as belonging to the match fragmentation pattern. In one or more embodiments, the match fragmentation pattern can be selected by the device based on an entity defined characteristic such as, for example, number of ion peaks matched. In one or more embodiments described in further detail below, the match fragmentation pattern can be selected by an annotation machine learning model. As described above, in some cases, one or more ion peaks may be left un-annotated, thereby producing an incomplete identification of the sample.

The second logic 104 may remove the one or more annotated ion peaks from the fragmentation spectrum and re-submit the fragmentation spectrum (without the previously-annotated ion peaks) to the first logic 102 for annotation of one or more of remaining ion peaks of the fragmentation spectrum. By removing the previously annotated ion peaks, the fragmentation spectrum can be focused to only include the ion peaks that have not already been matched to a known fragmentation pattern and, thus a known structure. This enables the subsequent search and annotation step to be focused on the previously un-annotated ion peaks, thereby enabling identification of additional molecular structures within the sample. In one or more embodiments, the second logic can determine if one or more defined search criteria have been met. For example, if a defined number of annotation iterations or loops have been executed, the second logic 104 can end the looping annotation process. In another example, if the defined search criteria specifies that all ion peaks are to be annotated, the second logic 104 can loop through the process of removing annotated ion peaks from the fragmentation spectrum and re-submitting the fragmentation spectrum to the first logic 102 until all ion peaks have been annotated. In a further example, the defined search criteria can comprise a similarity metric based on a comparison between the fragmentation spectrum of the unknown sample and the fragmentation spectrum of a known sample or compound. For example, a scoring algorithm can be used to compare peak mass-to-charge ratios and intensities between the fragmentation spectrum and the spectrum of the known sample. If the score does not reach a defined threshold, then looping can continue. Accordingly, the scientific instrument module 100 can facilitate looped spectral searches for residual ions within a fragmentation spectrum.

FIG. 2 is a flow diagram of a computer-implemented method 200 in accordance with one or more embodiments described herein. The operations of the computer-implemented method 200 may be used in any suitable setting to perform any suitable operations (e.g., can be performed by or used in conjunction with any of the various modules, computing devices, or graphical user interfaces described with respect to of FIGS. 1, 7, 8, 9, and 10). Operations are illustrated once each and in a particular order in FIG. 2, but the operations may be reordered or repeated as desired and appropriate (e.g., different operations performed may be performed in parallel, as suitable).

At 202, first operations may be performed. For example, the first logic 102 of scientific instrument module 100 may perform the operations of 202. The first operations may include annotating one or more ion peaks of a fragmentation spectrum.

At 204, second operations may be performed. For example, the second logic 104 of scientific instrument module 100 may perform the operations of 204. The second operations may include removing the one or more annotated ion peaks from the fragmentation spectrum.

At 206, third operations may be performed. For example, the first logic 102 and/ or second logic 104 may perform the operations of 206. The third operations may include annotating one or more remaining ion peaks of the fragmentation spectrum. Accordingly, the computer-implemented method 200 can facilitate looped searching of residual ion peaks in accordance with one or more embodiments described herein.

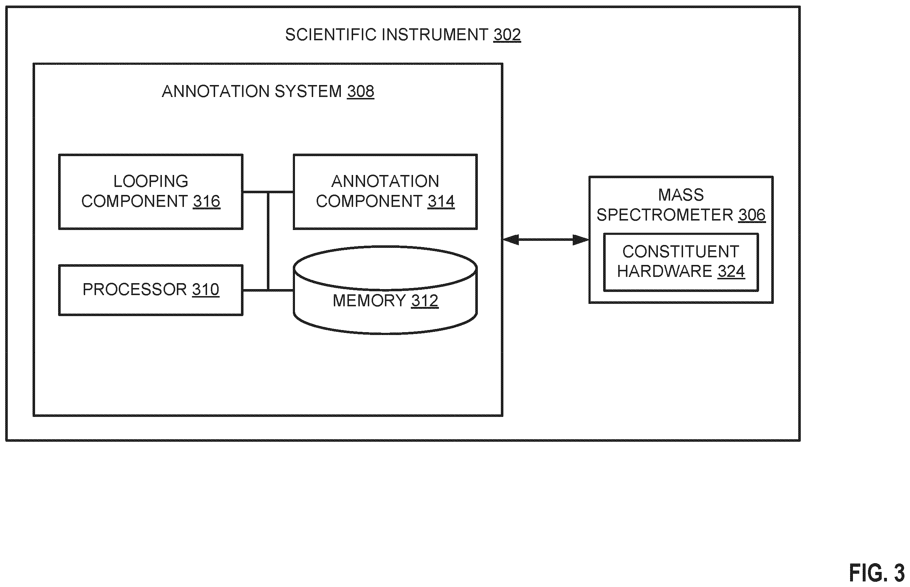

FIG. 3 illustrates a block diagram of an example, non-limiting scientific instrument that can facilitate looped spectral searches of residual ion peaks in accordance with one or more embodiments described herein. As shown, scientific instrument 302 can comprise a mass spectrometer 306.

In various aspects, the mass spectrometer 306 can be any suitable mass spectrometer. In various instances, the mass spectrometer 306 can comprise any suitable constituent hardware 324 for measuring ion spectra of analytical samples. In various cases, the ion beam emitter can receive a compositional part of the analytical sample and can ionize that compositional part into an ion beam. The ion beam emitter can facilitate this via any suitable ionization or fragmentation technique, such as electron ionization, chemical ionization, matrix assisted laser desorption ionization, electrospray ionization, photoionization, or inductively coupled plasma ionization, any of which can be implemented in a vacuum or at atmospheric pressure. In various aspects, the ion optics equipment can channel or steer the ion beam produced by the ion beam emitter through the mass analyzer and to the ion detector. Non-limiting examples of such ion optics equipment can include ion focusing lenses, ion guides, or ion deflectors. In various instances, the mass analyzer can separate or sort whatever ions are present in the ion beam according to their mass-to-charge ratios. Non-limiting examples of the mass analyzer can include quadrupole mass analyzers, time-of-flight mass analyzers, magnetic sector mass analyzers, electrostatic sector mass analyzers, quadrupole ion trap mass analyzers, orbitrap mass analyzer, asymmetric track lossless mass analyzer, or ion cyclotron resonance mass analyzers. In various cases, the ion detector can electronically detect or measure the relative abundances of whatever ions strike it. Non-limiting examples of the ion detector can include electron multiplier ion detectors, photomultiplier tubes, microchannel plate detectors, image charge detectors, or Faraday cup ion detectors.

In any case, when given an analytical sample, the mass spectrometer 306 can produce an ion spectrum plotting relative abundance against mass-to-chare ratio (e.g., fragmentation spectra) of the various ions of the analytical sample.

In various embodiments, the scientific instrument 302 can comprise an annotation system 308. In various cases the annotation system 308 can facilitate looped spectral searches for residual ions on the fragmentation spectra produced by mass spectrometer 306.

In various aspects, the annotation system 308 can comprise a processor 310 (e.g., computer processing unit, microprocessor) and a non-transitory computer-readable memory 312 that is operably or operatively or communicatively connected or coupled to the processor 310. The non-transitory computer-readable memory 312 can store computer-executable instructions which, upon execution by the processor 310, can cause the processor 310 or other components of the annotation system 308 (e.g., annotation component 314, looping component 316) to perform one or more acts. In various embodiments, the non-transitory computer-readable memory 312 can store computer-executable components (e.g., annotation component 314, looping component 316), and the processor 310 can execute the computer-executable components.

In various embodiments, the annotation system 308 can comprise an annotation component 314. In various aspects, as described herein, the annotation component 314 can annotate one or more ion peaks of a fragmentation spectrum produced by mass spectrometer 306 (as described above in relation to the first logic 102) to generate an annotated portion of a fragmentation spectrum. As described above, the annotation process can comprise matching, by the device, one or more ion peaks of the fragmentation spectrum to one or more ion peaks of one or more possible match fragmentation patterns stored in a pattern library, selecting, by the device, a match fragmentation pattern from the one or more possible match fragmentation patterns, and marking, by the device, the one or more matched ion peaks of the fragmentation spectrum as belonging to the match fragmentation pattern.

In one or more embodiments, the match fragmentation pattern can be selected from the one or more possible match fragmentation patterns based on a scoring algorithm that compares factors such as ion intensities. In one or more embodiments, the match fragmentation pattern can be selected based on one or more matching criteria such as, the number of matching ion peaks, and/or a historical relationship between previously annotated molecular substructures and the molecular structure of the one or more possible match fragmentation patterns. For example, if one or more previously removed ion peaks were annotated as belonging to a first molecular substructure, a historical relationship shows that molecular compounds with the first molecular substructure are likely to further comprise a second molecular substructure, and the second molecular substructure is identified as one of the possible match fragmentation patterns, then the fragmentation pattern corresponding to the second molecular substructure can be selected as the match fragmentation pattern. In one or more embodiments the historical relationship can be determined based on known fragmentation patterns with the pattern library, and a percentage as specified by an entity. For example, a historical relationship can be identified between the first molecular substructure and the second molecular substructure if a percentage of known fragmentation patterns containing the first molecular substructure also contain the second molecular substructure, wherein the percentage is defined by an entity. In another example, a historical relationship can be identified if there is a specified number of fragmentation patterns containing both the first molecular substructure and the second molecular substructure, wherein the specified number is defined by an entity.

In various embodiments, the annotation system 308 can comprise a looping component 316. In various aspects, the looping component 316 can remove the one or more ion peaks annotated by annotation component 314 from the fragmentation spectrum, determine if a defined search criteria has been met, and in response to the defined search criteria not being met, re-submit the fragmentation spectrum to the annotation component 314 for one or more addition annotation rounds or loops (as described above in relation to the second logic 104). In one or more embodiments, once annotation is complete, the final annotated fragmentation spectrum can be added to the pattern library to facilitate future identification of the same sample type.

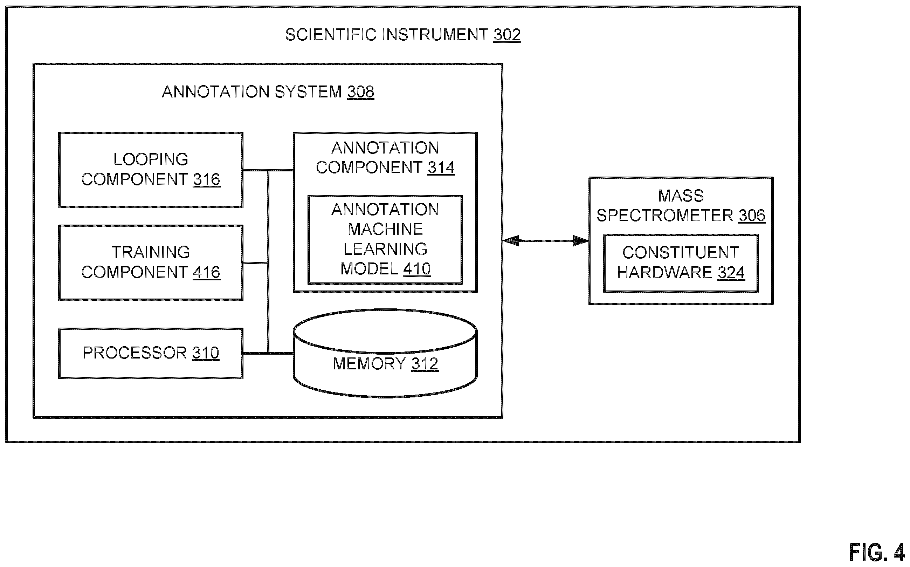

FIG. 4 illustrates a block diagram of an example, non-limiting scientific instrument that can facilitate looped spectral searches of residual ion peaks in accordance with one or more embodiments described herein. As shown, scientific instrument 302 can comprise a mass spectrometer 306 and annotation system 308 as described above in relation to FIG. 3. Annotation system 308 of FIG. 4 can further comprise annotation machine learning model 410 and training component 416. In one or more embodiments, training component 416 can train annotation machine learning model 410 to select the match fragmentation pattern from the one or more possible match fragmentation patterns, wherein the training comprises, generating, by the annotation machine learning model, an annotated fragmentation spectrum of a known sample, comparing, by training component 416, the annotated fragmentation spectrum to the fragmentation pattern of the known sample, and updating, by the training component 416, annotation machine learning model 410 based on the results of the comparison. In this manner, annotation machine learning model 410 can learn associations between molecular substructures of known samples, thereby enabling annotation machine learning model 410 to accurately select match fragmentation patterns from possible match fragmentation patterns in order to further automate the annotation of unknown samples.

According to some embodiments, annotation machine learning model 410 can employ automated learning and reasoning procedures (e.g., the use of explicitly and/or implicitly trained statistical classifiers) in connection with performing inference and/or probabilistic determinations and/or statistical-based determinations in accordance with one or more aspects described herein.

For example, annotation machine learning model 410 can employ principles of probabilistic and decision theoretic inference to determine one or more responses based on information retained in a knowledge source database. In various embodiments, annotation machine learning model 410 can employ a knowledge source database comprising known fragmentation patterns and the molecular structures/substructures associated with the known fragmentation patterns. Additionally, or alternatively, annotation machine learning model 410 can rely on predictive models constructed using machine learning and/or automated learning procedures. Logic-centric inference can also be employed separately or in conjunction with probabilistic methods. For example, decision tree learning can be utilized to map observations about data retained in a knowledge source database to derive an appropriate annotation for one or more ion peaks of a fragmentation spectrum.

As used herein, the term "inference" refers generally to the process of reasoning about or inferring states of the system, a component, a module, the environment, and/or assessments from one or more observations captured through events, reports, data, and/or through other forms of communication. Inference can be employed to identify a specific context or action, or can generate a probability distribution over states, for example. The inference can be probabilistic. For example, computation of a probability distribution over states of interest can be based on a consideration of data and/or events. The inference can also refer to techniques employed for composing higher-level events from one or more events and/or data. Such inference can result in the construction of new events and/or actions from one or more observed events and/or stored event data, whether or not the events are correlated in close temporal proximity, and whether the events and/or data come from one or several events and/or data sources. Various classification schemes and/or systems (e.g., support vector machines, neural networks, logic-centric production systems, Bayesian belief networks, fuzzy logic, data fusion engines, and so on) can be employed in connection with performing automatic and/or inferred action in connection with the disclosed aspects. Furthermore, the inference processes can be based on stochastic or deterministic methods, such as random sampling, Monte Carlo Tree Search, and so on.

The various aspects can employ various artificial intelligence-based schemes for carrying out various aspects thereof. For example, a process of selecting a match fragmentation pattern from one or more possible match fragmentation patterns, without interaction from the target entity, which can be enabled through an automatic classifier system and process.

A classifier is a function that maps an input attribute vector, x = (x1, x2, x3, x4, xn), to a confidence that the input belongs to a class. In other words, f(x) = confidence(class). Such classification can employ a probabilistic and/or statistical-based analysis (e.g., factoring into the analysis utilities and costs) to prognose or infer an action that should be employed to make a determination. The determination can include, but is not limited to, what match fragmentation pattern should be selected from one or more possible match fragmentation patterns.

A support vector machine (SVM) is an example of a classifier that can be employed. The SVM operates by finding a hypersurface in the space of possible inputs, which hypersurface attempts to split the triggering criteria from the non-triggering events. Intuitively, this makes the classification correct for testing data that can be similar, but not necessarily identical to training data. Other directed and undirected model classification approaches (e.g., naïve Bayes, Bayesian networks, decision trees, neural networks, fuzzy logic models, and probabilistic classification models) providing different patterns of independence can be employed. Classification as used herein, can be inclusive of statistical regression that is utilized to develop models of priority.

One or more aspects can employ classifiers that are explicitly trained (e.g., through a generic training data) as well as classifiers that are implicitly trained (e.g., by observing and recording target entity behavior, by receiving extrinsic information, and so on). For example, SVM's can be configured through a learning phase or a training phase within a classifier constructor and feature selection module. Thus, a classifier(s) can be used to automatically learn and perform a number of functions, including but not limited to selection of match fragmentation patterns from one or more possible match fragmentation patterns. Furthermore, one or more aspects can employ machine learning models that are trained utilizing reinforcement learning. For example, penalty/reward scores can be assigned for various outputs generated by annotation machine learning model 410 based on defined entity preferences. Accordingly, annotation machine learning model 410 can learn via selecting options with lower penalties and/or higher rewards in order to reduce an overall penalty score and/or increase an overall reward score. For example, training component 416 can assign a penalty/reward score to the annotations generated by annotation machine learning model 410 based on comparison of the annotations to the known fragmentation spectrum of the sample used during training.

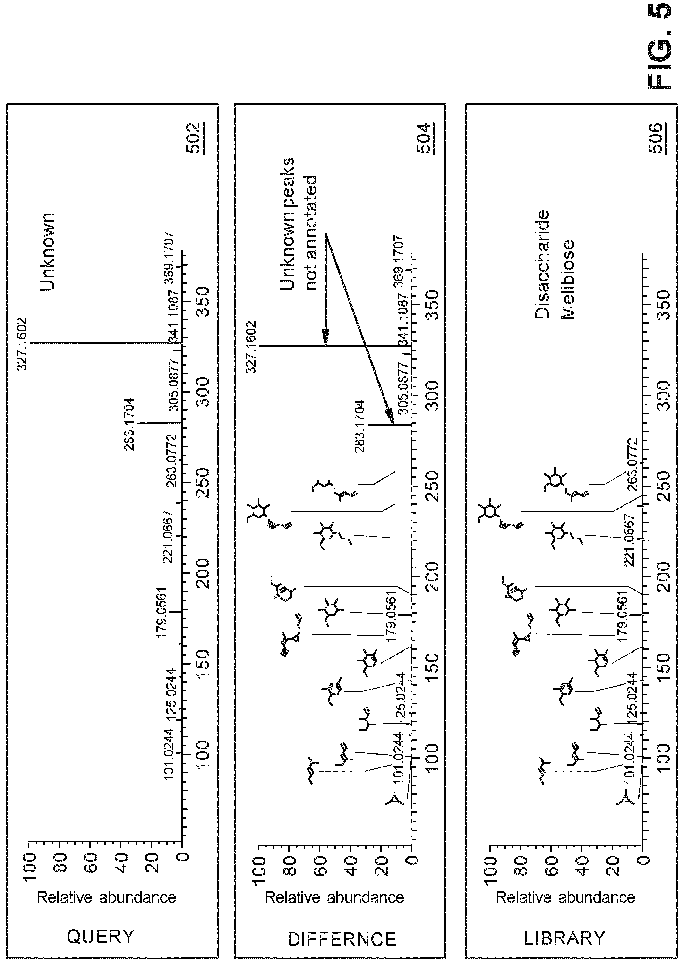

FIG. 5 illustrates a flow diagram of a single spectral search in accordance with one or more embodiments described herein.

As shown, block 502 is a fragmentation spectrum of a sample showing ion peaks as determined by a mass spectrometer. Block 506 is a fragmentation pattern of a known structure, in this case Disaccharide Melibiose, comprising one or more ion peaks matching one or more ion peaks of block 502. Accordingly, as described above in relation to FIG. 1, the one or more matching peaks can be annotated based on the library structure. However, as shown in block 504, this leaves some ion peaks unknown/un-annotated. Existing spectral search techniques end the analysis process at this stage leaving one or more ion peaks un-annotated and thus at least part of the structure of the sample, un-identified.

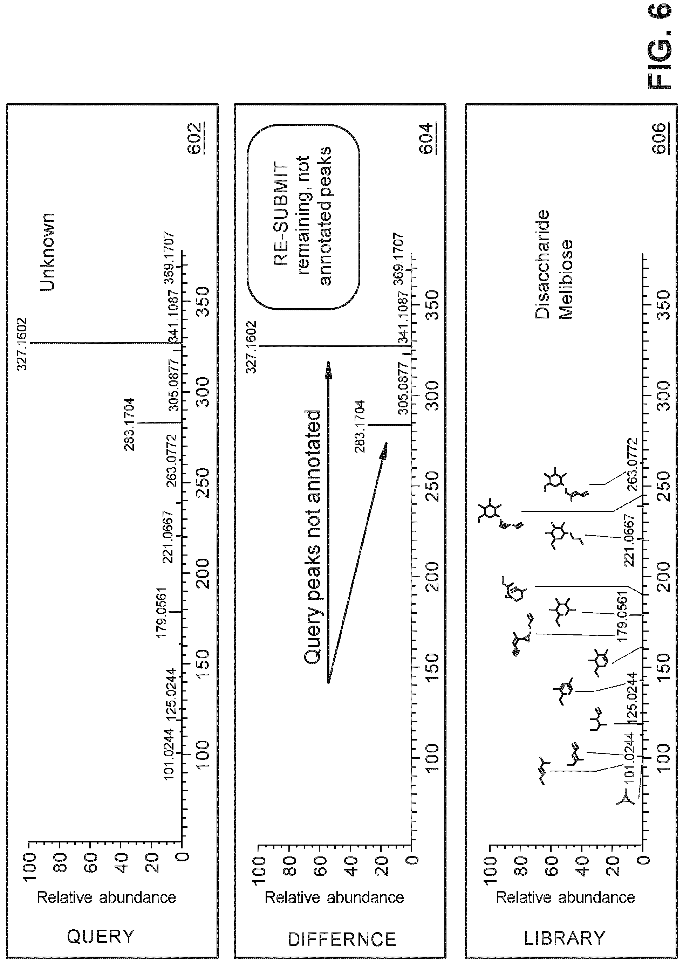

FIG. 6 illustrates a flow diagram of a spectral search with a single loop for a search of residual ions in accordance with one or more embodiments described herein.

As shown, block 602 is a fragmentation spectrum of a sample showing ion peaks as determined by a mass spectrometer. Block 606 is a fragmentation pattern of a known structure, in this case Disaccharide Melibiose, comprising one or more ion peaks matching one or more ion peaks of block 602. Accordingly, as described above in relation to FIG. 1, the one or more matching peaks can be annotated based on the library structure. As described above in relation to FIG. 5, one or more ion peaks are left un-annotated as shown in block 604. Furthermore, as described above in relation to FIG. 1, the annotated ion peaks (e.g., those that are annotated as being components of Disaccharide Melibiose) can be removed (as described in relation to the second logic 104) and the fragmentation spectrum with the annotated ion peaks removed (as shown in block 604) can then be re-submitted for another loop or iteration of annotation in order to annotate one or more of the remaining ion peaks.

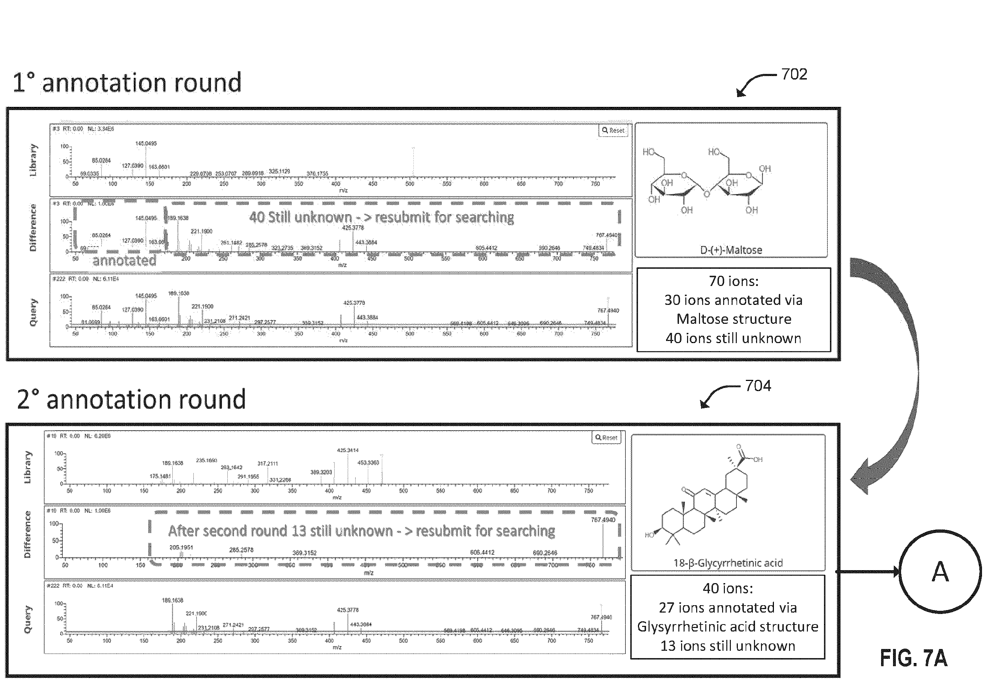

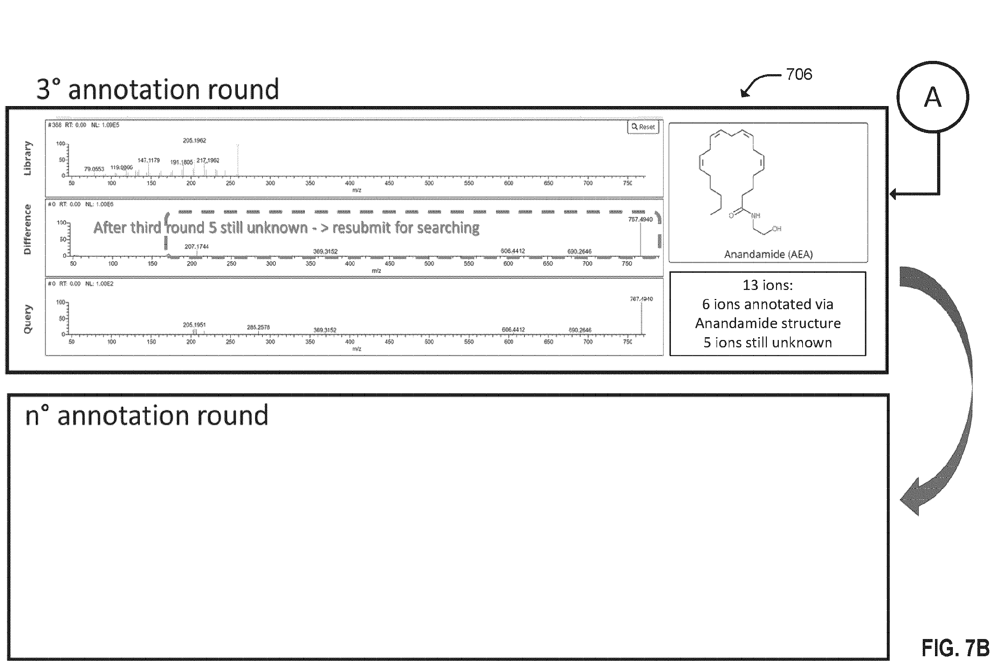

FIGS. 7A and 7B illustrate a flow diagram of multiple search loops of residual ions in accordance with one or more embodiments described herein.

At step 702, a fragmentation spectrum of a sample is received comprising 100 ion peaks. As described above in relation to FIG. 1, one or more ion peaks can be annotated (e.g., using the first logic 102) to generate an annotated portion of a fragmentation spectrum. As shown, 70 of the ion peaks are annotated as being components of a Maltose structure. The annotated ion peaks can then be removed from the fragmentation sample (e.g., using the second logic 104) and the fragmentation sample is resubmitted for a second round of annotations (e.g., step 704). At step 704, 27 of the remaining 40 ion peaks are annotated as components of a Glycyrrhetinic acid structure. The 27 annotated ion peaks can then be removed, and the remaining 13 un-annotated ion peaks can be re-submitted for a third round/iteration of annotation (e.g., step 706). At step 706, 6 of the remaining ion peaks are annotated as components of an Anandamide structure. The 6 annotated ion peaks are removed, and the remaining 5 ion peaks are re-submitted for one or more further rounds of annotation. It should be appreciated that the looping search of residual ions can continue until a set number of ion peaks are annotated, until a set percentage of the total number of ion peaks are annotated, until a defined number of loops are executed, until a defined amount of time has passed, or another defined search criteria is met.

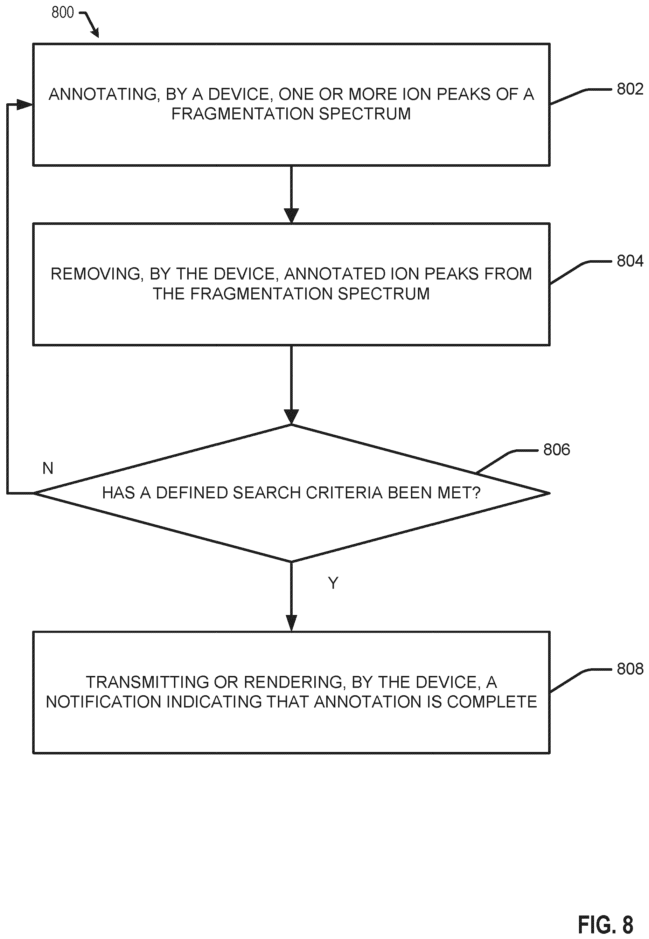

FIG. 8 illustrates a flow diagram of an example, non-limiting computer-implemented method 800 that can facilitate looped annotation of fragmentation spectra of unknown samples in accordance with one or more embodiments described herein.

In various cases, annotation system 308 can facilitate the computer-implemented method 800. In various embodiments, act 802 can comprise, annotating, by a device (e.g., via annotation component 314) one or more ion peaks of a fragmentation spectrum produced by a mass spectrometer (e.g., mass spectrometer 306). For example, as described above in reference to FIGS. 1-4, the annotating can comprise matching, by the device one or more ion peaks of the fragmentation spectrum to one or more ion peaks of one or more possible match fragmentation patterns stored in a pattern library, selecting, by the device, a match fragmentation pattern from the one or more possible match fragmentation patterns, and marking, by the device, the one or more matched ion peaks of the fragmentation spectrum as belonging to a molecular substructure associated with the match fragmentation pattern. As described above in relation to FIGS. 1-4, in one or more embodiments, the selection of the match fragmentation pattern can be based at least in part on a stored relationship between the match fragmentation pattern and a match fragmentation pattern previously utilized to annotated one or more ion peaks of the fragmentation spectrum. For example, given a historical correlation between a first match fragmentation pattern and a second match fragmentation pattern, and that the first match fragmentation pattern was selected during a previous iteration/loop of annotation, the second match fragmentation pattern can be selected, given that the one or more possible match fragmentation patterns comprises the second match fragmentation pattern.

In various embodiments, act 804 can comprise removing, by the device (e.g., annotation component 314) annotated ion peaks from the fragmentation spectrum.

In various embodiments, act 806 can comprise checking, by the device (e.g., looping component 316) if a defined search criteria has been met. For example, as described above in reference to FIGS. 1-4, the define search criteria can comprise at least one of a defined number of annotation loops/iterations, a defined number of ion peaks annotated, a defined percentage of total ion peaks being annotated, an amount of elapsed search time, or other defined search criteria. If the defined search criteria have been met or exceeded, method 800 can proceed to act 808. If the defined search criteria have not been met, method 800 can return to act 802 for one or more additional iterations/loops of annotation.

In various embodiments, act 808 can comprise transmitting or rendering, by the device, a notification indicating that annotation is complete. In one or more embodiments, the notification can comprise the annotations portions of the fragmentation spectrum. For example, the completed annotated fragmentation spectrum can comprise the annotations generated during each iteration/round of annotation. In one or more embodiments, the completed annotations can be stored as a new fragmentation pattern in a pattern library to enable identification of the same type of sample in the future.

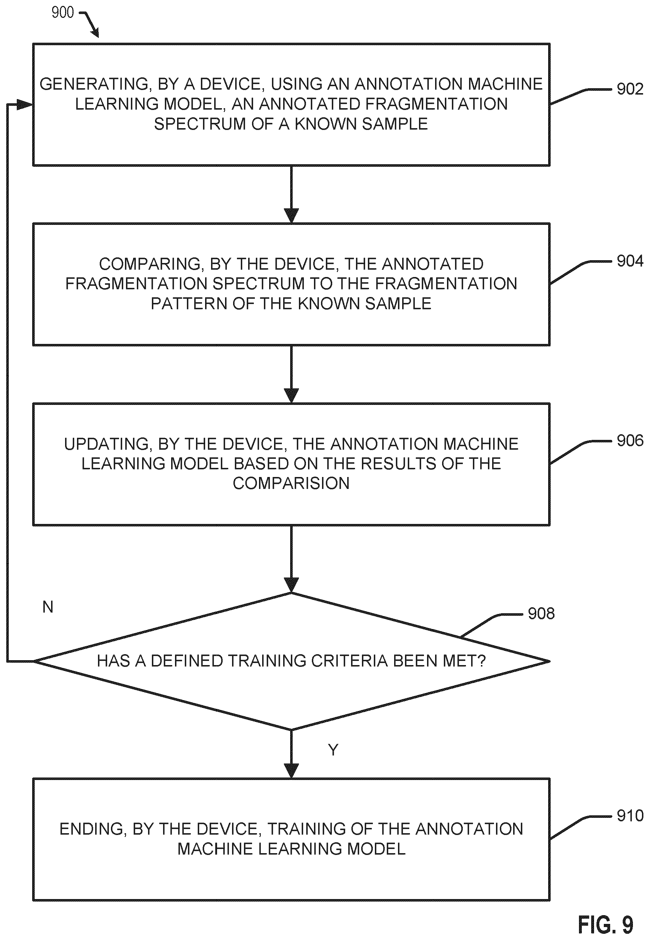

FIG. 9 illustrates a flow diagram of an example, non-limiting, computer-implemented method 900 that can facilitate training of an annotation machine learning model in accordance with one or more embodiments described herein.

In various embodiments, act 902 can comprise generating, by a device (e.g., annotation component 314), using an annotation machine learning model (e.g., annotation machine learning model 410) an annotated fragmentation spectrum of a known sample. For example, annotation machine learning model 410 can be given a fragmentation spectrum of a known sample and can annotate the fragmentation spectrum as described above in relation to FIG. 8, wherein annotation machine learning model 410 selects match fragmentation patterns from the one or more possible match fragmentation patterns, and wherein the complete fragmentation pattern of the known sample has been removed from the pattern library.

In various embodiments, act 904 can comprise comparing, by the device (e.g., training component 416), the annotated fragmentation spectrum produced by annotation machine learning model 410 to the complete fragmentation pattern of the known sample. For example, training component 416 can compare the annotated fragmentation pattern to the known fragmentation pattern to determine if annotation machine learning model 410 correctly identified the molecular subcomponents with the known sample.

In various embodiments, act 906 can comprise updating, by the device (e.g., training component 416) the annotation machine learning model 410 based on the results of the comparison. For example, if an embodiment, if annotation machine learning model correctly identified one or more molecular subcomponents of the known sample, training component 416 can assign a reward score based on the correctly identified molecular subcomponents. If the annotation machine learning model incorrectly identifies one or more molecular subcomponents of the sample (e.g., selects a possible match fragmentation pattern that is not present in the sample, or does not select a possible match fragmentation pattern that is present in the sample) training component 416 can assign a penalty score. Accordingly, annotation machine learning model can be trained using an objective function, wherein annotation machine learning model 410 attempts to maximize the reward score and/or minimize the penalty score.

In various embodiments, act 908 can comprise checking, by the device (e.g., training component 416) if a defined training criteria has been met. In one or more embodiments, the defined training criteria can comprise at least one of a defined amount of elapsed training time, a defined number of training cycles, iterating through all known samples in a training data set, achieving a defined accuracy level, and/or another criteria utilized to determine when training of annotation machine learning model 410 is complete. In response to the defined training criteria being met, method 900 can proceed to act 910 and end training of annotation machine learning model 410. In response to the defined training criteria not being met, method 900 can return to act 902, and annotation machine learning model can be trained on one or more additional known samples within a training data set.

An advantage of the systems, and/or of corresponding computer-implemented methods and/or computer program products described herein can be the ability to repeatedly annotate ion peaks within a fragmentation spectrum, allowing for the identification of multiple molecules or structures within a fragmentation spectrum. This enabled greater annotation coverage, and thus a more detailed analysis of unknown samples when compared to existing approaches.

The scientific instrument systems, methods, or techniques disclosed herein may include interactions with a human user (e.g., via a user local computing device 1220 discussed herein with reference to FIG. 12). These interactions may include providing information to the user (e.g., information regarding the operation of a scientific instrument such as the scientific instrument 1210 of FIG. 12, information regarding a sample being analyzed or other test or measurement performed by a scientific instrument, information retrieved from a local or remote database, or other information) or providing an option for a user to input commands (e.g., to control the operation of a scientific instrument such as the scientific instrument 1210 of FIG. 12, or to control the analysis of data generated by a scientific instrument), queries (e.g., to a local or remote database), or other information. In some embodiments, these interactions may be performed through a graphical user interface (GUI) that includes a visual display on a display device (e.g., a display device 1110 discussed herein with reference to FIG. 11) that provides outputs to the user and/or prompts the user to provide inputs (e.g., via one or more input devices, such as a keyboard, mouse, trackpad, or touchscreen, included in other I/O devices 1112 discussed herein with reference to FIG. 11). The scientific instrument systems, methods, or techniques disclosed herein may include any suitable GUIs for interaction with a user.

FIG. 10 depicts an example graphical user interface 10000 (hereafter "GUI 10000") that can be used in the performance of some or all of the support methods or techniques disclosed herein, in accordance with various embodiments. In various aspects, the GUI 10000 can be provided on any suitable electronic display (e.g., a display device 1110 discussed herein with reference to FIG. 11) of a computing device (e.g., a computing device 1100 discussed herein with reference to FIG. 11) of a scientific instrument support system (e.g., a scientific instrument support system 1200 discussed herein with reference to FIG. 12), and a user or technician can interact with the GUI 1000 using any suitable input device (e.g., any of other I/O devices 1112 discussed herein with reference to FIG. 11) and input technique (e.g., movement of a cursor, motion capture, facial recognition, gesture detection, voice recognition, actuation of buttons).

The GUI 1100 can include a data display region 1102, a data analysis region 1104, a scientific instrument control region 1106, and a setting region 1108. The particular number and arrangement of regions depicted in FIG. 11 is merely illustrative, and any number and arrangement of regions, including any desired features, can be included in other embodiments of the GUI 1100.

The data display region 1102 can display data generated by a scientific instrument (e.g., a scientific instrument 1210 discussed herein with reference to FIG. 12). For example, the data display region 10002 can display any of the one or more measured fragmentation spectra and/or annotated ion peaks.

The data analysis region 1004 can display any suitable data analysis results (e.g., the results of analyzing the data illustrated in the data display region 1002 or other data). For example, the data analysis region 1004 can display any electronic notifications that are generated by the annotation component 314 and/or the looping component 316. In some embodiments, the data display region 1102 and the data analysis region 1104 can be combined in the GUI 1000 (e.g., to include both data output from a scientific instrument and some analysis of the data in a common graph or region).

The scientific instrument control region 1006 can include options that allow a user or technician to control a scientific instrument (e.g., the scientific instrument 1210 discussed herein with reference to FIG. 12). For example, the scientific instrument control region 1006 can include configurable parameters that govern operation of such scientific instrument (e.g., configurable parameters that govern voltages or currents of the scientific instrument, that govern interior temperatures of the scientific instrument, or that govern fluid flow rates of the scientific instrument).

The setting region 1008 can include options that allow a user or technician to control any features or functions of the GUI 1000 (or of other GUIs) or to perform common computing operations with respect to the data display region 1002 and the data analysis region 1004 (e.g., saving data on a storage device, such as the storage device 1104 discussed herein with reference to FIG. 11, sending data to another user, labeling data).

As noted above, the scientific instrument module 100 can be implemented by one or more computing devices. FIG. 11 is a block diagram of a computing device 1100 that can perform some or all of the scientific instrument methods or techniques disclosed herein, in accordance with various embodiments. In some embodiments, the scientific instrument module 100 can be implemented by a single instance of the computing device 1100 or by multiple instances of the computing device 1100. Further, as discussed below, the computing device 1100 (or multiple instances thereof) that implements the scientific instrument module 100 can be part of one or more of a scientific instrument 1210, a user local computing device 1220, a service local computing device 1230, or a remote computing device 1240 of FIG. 12.

The computing device 1100 is illustrated as having a number of components, but any one or more of these components can be omitted or duplicated, as suitable for the application and setting. In some embodiments, some or all of the components included in the computing device 1100 can be attached to one or more motherboards and enclosed in a housing (e.g., including plastic, metal, or other materials). In some embodiments, some these components can be fabricated onto a single system-on-a-chip (SoC) (e.g., an SoC may include one or more instances of a processing device 1102 and one or more instances of a storage device 1104). Additionally, in various embodiments, the computing device 1100 can omit one or more of the components illustrated in FIG. 11, but can include interface circuitry (not shown) for coupling to the one or more omitted components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface) . For example, the computing device 1100 can omit a display device 1110, but can include display device interface circuitry (e.g., a connector and driver circuitry) to which a display device 1110 can be coupled.

The computing device 1100 can include a processing device 1102 (e.g., one or more processing devices). As used herein, the term "processing device" can refer to any device or portion of a device that processes electronic data from registers or memory to transform that electronic data into other electronic data that may be stored in registers or memories. The processing device 1102 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 1100 can include a storage device 1104 (e.g., one or more storage devices). The storage device 1104 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 1104 can include memory that shares a die with a processing device 1102. In such an embodiment, the memory may be used as cache memory and may include embedded dynamic random access memory (eDRAM) or spin transfer torque magnetic random access memory (STT-MRAM), for example. In some embodiments, the storage device 1104 can include non-transitory computer readable media having instructions thereon that, when executed by one or more processing devices (e.g., the processing device 1102), cause the computing device 1100 to perform any appropriate ones of or portions of the methods disclosed herein.

The computing device 1100 can include an interface device 1106 (e.g., one or more instances of the interface device 1106). The interface device 1106 can include one or more communication chips, connectors, or other hardware and software to govern communications between the computing device 1100 and other computing devices. For example, the interface device 1106 can include circuitry for managing wireless communications for the transfer of data to and from the computing device 1100. The term "wireless" and its derivatives may be used to describe circuits, devices, systems, methods, techniques, or communications channels that may communicate data through the use of modulated electromagnetic radiation through a nonsolid medium. The term does not imply that the associated devices do not contain any wires, although in some embodiments they might not. Circuitry included in the interface device 1106 for managing wireless communications may implement any of a number of wireless standards or protocols, including but not limited to Institute for Electrical and Electronic Engineers (IEEE) standards including Wi-Fi (IEEE 802.11 family), IEEE 802.8 standards (e.g., IEEE 802.8-2005 Amendment), Long-Term Evolution (LTE) project along with any amendments, updates, and/or revisions (e.g., advanced LTE project, ultra mobile broadband (UMB) project (also referred to as "3GPP2")). In some embodiments, circuitry included in the interface device 1106 for managing wireless communications can operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 1106 for managing wireless communications can operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 1106 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 1106 may include one or more antennas (e.g., one or more antenna arrays) to receipt and/or transmission of wireless communications.

In some embodiments, the interface device 1106 can include circuitry for managing wired communications, such as electrical, optical, or any other suitable communication protocols. For example, the interface device 1106 can include circuitry to support communications in accordance with Ethernet technologies. In some embodiments, the interface device 1106 can support both wireless and wired communication, or can support multiple wired communication protocols or multiple wireless communication protocols. For example, a first set of circuitry of the interface device 1106 may be dedicated to shorter-range wireless communications such as Wi-Fi or Bluetooth, and a second set of circuitry of the interface device 1106 may be dedicated to longer-range wireless communications such as global positioning system (GPS), EDGE, GPRS, CDMA, WiMAX, LTE, EV-DO, or others. In some embodiments, a first set of circuitry of the interface device 1106 can be dedicated to wireless communications, and a second set of circuitry of the interface device 1106 can be dedicated to wired communications.

The computing device 1100 can include battery/power circuitry 1108. The battery/power circuitry 1108 can include one or more energy storage devices (e.g., batteries or capacitors) or circuitry for coupling components of the computing device 1100 to an energy source separate from the computing device 1100 (e.g., alternating current line power).

The computing device 1100 can include a display device 1110 (e.g., multiple display devices). The display device 1110 can include any visual indicators, such as a heads-up display, a computer monitor, a projector, a touchscreen display, a liquid crystal display (LCD), a light-emitting diode display, or a flat panel display.

The computing device 1100 can include other input/output (I/O) devices 1112. The other I/O devices 1112 can include one or more audio output devices (e.g., speakers, headsets, earbuds, alarms), one or more audio input devices (e.g., microphones or microphone arrays), location devices (e.g., GPS devices in communication with a satellite-based system to receive a location of the computing device 1100), audio codecs, video codecs, printers, sensors (e.g., thermocouples or other temperature sensors, humidity sensors, pressure sensors, vibration sensors, accelerometers, gyroscopes), image capture devices such as cameras, keyboards, cursor control devices such as a mouse, a stylus, a trackball, or a touchpad, bar code readers, Quick Response (OR) code readers, or radio frequency identification (RFID) readers, for example.

The computing device 1100 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer), a desktop computing device, or a server computing device or other networked computing component.

One or more computing devices implementing any of the scientific instrument modules, methods, or techniques disclosed herein may be part of a scientific instrument support system. FIG. 12 is a block diagram of an example scientific instrument support system 1200 in which some or all of the scientific instrument support methods disclosed herein may be performed, in accordance with various embodiments. The scientific instrument modules, methods, or techniques disclosed herein (e.g., the scientific instrument module 100, the computer-implemented method 200, the annotation system 308) can be implemented by one or more of a scientific instrument 1210, a user local computing device 1220, a service local computing device 1230, or a remote computing device 1240 of the scientific instrument support system 1200.

Any of the scientific instrument 1210, the user local computing device 1220, the service local computing device 1230, or the remote computing device 1240 can include any of the embodiments of the computing device 1100, and any of the scientific instrument 1210, the user local computing device 1220, the service local computing device 1230, or the remote computing device 1240 can take the form of any appropriate ones of the embodiments of the computing device 1100.

The scientific instrument 1210, the user local computing device 1220, the service local computing device 1230, or the remote computing device 1240 may each include a processing device 1202, a storage device 1204, and an interface device 1206. The processing device 1202 may take any suitable form, including any form of the processing device 1102, and the processing devices 1202 included in different ones of the scientific instrument 1210, the user local computing device 1220, the service local computing device 1230, or the remote computing device 1240 may take the same form or different forms. The storage device 1204 may take any suitable form, including any form of the storage device 1104, and the storage devices 1204 included in different ones of the scientific instrument 1210, the user local computing device 1220, the service local computing device 1230, or the remote computing device 1240 may take the same form or different forms. The interface device 1206 may take any suitable form, including any form of the interface device 1106, and the interface devices 1206 included in different ones of the scientific instrument 1210, the user local computing device 1220, the service local computing device 1230, or the remote computing device 1240 may take the same form or different forms.

The scientific instrument 1210, the user local computing device 1220, the service local computing device 1230, and the remote computing device 1240 can be in communication with other elements of the scientific instrument support system 1200 via communication pathways 1208. The communication pathways 1208 may communicatively couple the interface devices 1206 of different ones of the elements of the scientific instrument support system 1200, as shown, and may be wired or wireless communication pathways (e.g., in accordance with any of the communication techniques discussed herein with reference to the interface device 1106). The particular scientific instrument support system 1200 depicted in FIG. 12 includes communication pathways between each pair of the scientific instrument 1210, the user local computing device 1220, the service local computing device 1230, and the remote computing device 1240, but this "fully connected" implementation is merely illustrative, and in various embodiments, various ones of the communication pathways 1208 may be absent. For example, in some embodiments, a service local computing device 1230 can lack a direct communication pathway 1208 between its interface device 1206 and the interface device 1206 of the scientific instrument 1210, but can instead communicate with the scientific instrument 1210 via the communication pathway 1208 between the service local computing device 1230 and the user local computing device 1220 and the communication pathway 1208 between the user local computing device 1220 and the scientific instrument 1210.

The scientific instrument 1210 may include any appropriate scientific instrument, such as the scientific instrument 302 .

The user local computing device 1220 can be a computing device (e.g., in accordance with any of the embodiments of the computing device 1100) that is local to a user of the scientific instrument 1210. In some embodiments, the user local computing device 1220 may also be local to the scientific instrument 1210, but this need not be the case; for example, a user local computing device 1220 that is in a user's home or office may be remote from, but in communication with, the scientific instrument 1210 so that the user may use the user local computing device 1220 to control or access data from the scientific instrument 1210. In some embodiments, the user local computing device 1220 may be a laptop, smartphone, or tablet device. In some embodiments the user local computing device 1220 can be a portable computing device.

The service local computing device 1230 can be a computing device (e.g., in accordance with any of the embodiments of the computing device 1100) that is local to an entity that services the scientific instrument 1210. For example, the service local computing device 1230 may be local to a manufacturer of the scientific instrument 1210 or to a third-party service company. In some embodiments, the service local computing device 1230 can communicate with the scientific instrument 1210, the user local computing device 1220, or the remote computing device 1240 (e.g., via a direct communication pathway 1208 or via multiple "indirect" communication pathways 1208, as discussed above) to receive data regarding the operation of the scientific instrument 1210, the user local computing device 1220, or the remote computing device 1240 (e.g., the results of self-tests of the scientific instrument 1210, calibration coefficients used by the scientific instrument 1210, the measurements of sensors associated with the scientific instrument 1210). In some embodiments, the service local computing device 1230 may communicate with the scientific instrument 1210, the user local computing device 1220, or the remote computing device 1240 (e.g., via a direct communication pathway 1208 or via multiple "indirect" communication pathways 1208, as discussed above) to transmit data to the scientific instrument 1210, the user local computing device 1220, or the remote computing device 1240 (e.g., to update programmed instructions, such as firmware, in the scientific instrument 1210, to initiate the performance of test or calibration sequences in the scientific instrument 1210, to update programmed instructions, such as software, in the user local computing device 1220 or the remote computing device 1240). A user of the scientific instrument 1210 can utilize the scientific instrument 1210 or the user local computing device 1220 to communicate with the service local computing device 1230 to report a problem with the scientific instrument 1210 or the user local computing device 1220, to request a visit from a technician to improve the operation of the scientific instrument 1210, to order consumables or replacement parts associated with the scientific instrument 1210, or for other purposes.

The remote computing device 1240 can be a computing device (e.g., in accordance with any of the embodiments of the computing device 1100 discussed herein) that is remote from the scientific instrument 1210 or from the user local computing device 1220. In some embodiments, the remote computing device 1240 can be included in a datacenter or other large-scale server environment. In some embodiments, the remote computing device 1240 may include network-attached storage (e.g., as part of the storage device 1204). The remote computing device 1240 can store data generated by the scientific instrument 1210, perform analyses of the data generated by the scientific instrument 1210 (e.g., in accordance with programmed instructions), facilitate communication between the user local computing device 1220 and the scientific instrument 1210, or facilitate communication between the service local computing device 1230 and the scientific instrument 1210.

In some embodiments, one or more of the elements of the scientific instrument support system 1200 illustrated in FIG. 12 can be omitted. Further, in some embodiments, multiple ones of various ones of the elements of the scientific instrument support system 1200 of FIG. 12 may be present. For example, a scientific instrument support system 1200 can include multiple user local computing devices 1220 (e.g., different user local computing devices 1220 associated with different users or in different locations). In another example, a scientific instrument support system 1200 may include multiple scientific instruments 1210, all in communication with service local computing device 1230 and/or a remote computing device 1240; in such an embodiment, the service local computing device 1230 may monitor these multiple scientific instruments 1210, and the service local computing device 1230 may cause updates or other information may be "broadcast" to multiple scientific instruments 1210 at the same time. Different ones of the scientific instruments 1210 in a scientific instrument support system 1200 can be located close to one another (e.g., in the same room) or farther from one another (e.g., on different floors of a building, in different buildings, in different cities, etc.). In some embodiments, a scientific instrument 1210 can be connected to an Internet-of-Things (IoT) stack that allows for command and control of the scientific instrument 1210 through a web-based application, a virtual or augmented reality application, a mobile application, or a desktop application. Any of these applications can be accessed by a user operating the user local computing device 1220 in communication with the scientific instrument 1210 by the intervening remote computing device 1240. In some embodiments, a scientific instrument 1210 may be sold by the manufacturer along with one or more associated user local computing devices 1220 as part of a local scientific instrument computing unit 1212.

In some embodiments, different ones of the scientific instruments 1210 included in a scientific instrument support system 1200 may be different types of scientific instruments 1210; for example, one scientific instrument 1210 may be a mass spectrometer, while another scientific instrument 1210 may be a chromatograph. In some such embodiments, the remote computing device 1240 or the user local computing device 1220 can combine data from different types of scientific instruments 1210 included in a scientific instrument support system 1200.

In various instances, machine learning algorithms or models can be implemented in any suitable way to facilitate any suitable aspects described herein. To facilitate some of the above-described machine learning aspects of various embodiments, consider the following discussion of artificial intelligence (Al). Various embodiments described herein can employ artificial intelligence to facilitate automating one or more features or functionalities. The components can employ various Al-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (z1, z2, z3, z4, zn), to a confidence that the input belongs to a class, as by f(z) = confidence(class). Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naïve Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

In order to provide additional context for various embodiments described herein, FIG. 10 and the following discussion are intended to provide a brief, general description of a suitable computing environment 1000 in which the various embodiments of the embodiment described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 13, the example environment 1300 for implementing various embodiments of the aspects described herein includes a computer 1302, the computer 1302 including a processing unit 1304, a system memory 1306 and a system bus 1308. The system bus 1308 couples system components including, but not limited to, the system memory 1306 to the processing unit 1304. The processing unit 1304 can be any of various commercially available processors. Dual microprocessors and other multi processor architectures can also be employed as the processing unit 1304.

The system bus 1308 can be any of several types of bus structure that can further interconnect to a memory bus (with or without a memory controller), a peripheral bus, and a local bus using any of a variety of commercially available bus architectures. The system memory 1306 includes ROM 1313 and RAM 1312. A basic input/output system (BIOS) can be stored in a non-volatile memory such as ROM, erasable programmable read only memory (EPROM), EEPROM, which BIOS contains the basic routines that help to transfer information between elements within the computer 1302, such as during startup. The RAM 1312 can also include a high-speed RAM such as static RAM for caching data.

The computer 1302 further includes an internal hard disk drive (HDD) 1314 (e.g., EIDE, SATA), one or more external storage devices 1316 (e.g., a magnetic floppy disk drive (FDD) 1316, a memory stick or flash drive reader, a memory card reader, etc.) and a drive 1320, e.g., such as a solid state drive, an optical disk drive, which can read or write from a disk 1322, such as a CD-ROM disc, a DVD, a BD, etc. Alternatively, where a solid state drive is involved, disk 1322 would not be included, unless separate. While the internal HDD 1314 is illustrated as located within the computer 1302, the internal HDD 1314 can also be configured for external use in a suitable chassis (not shown). Additionally, while not shown in environment 1300, a solid state drive (SSD) could be used in addition to, or in place of, an HDD 1314. The HDD 1314, external storage device(s) 1316 and drive 1320 can be connected to the system bus 1308 by an HDD interface 1324, an external storage interface 1326 and a drive interface 1328, respectively. The interface 1324 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 1302, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1312, including an operating system 1330, one or more application programs 1332, other program modules 1334 and program data 1336. All or portions of the operating system, applications, modules, or data can also be cached in the RAM 1312. The systems and methods described herein can be implemented utilizing various commercially available operating systems or combinations of operating systems.

Computer 1302 can optionally comprise emulation technologies. For example, a hypervisor (not shown) or other intermediary can emulate a hardware environment for operating system 1330, and the emulated hardware can optionally be different from the hardware illustrated in FIG. 13. In such an embodiment, operating system 1330 can comprise one virtual machine (VM) of multiple VMs hosted at computer 1302. Furthermore, operating system 1330 can provide runtime environments, such as the Java runtime environment or the .NET framework, for applications 1332. Runtime environments are consistent execution environments that allow applications 1332 to run on any operating system that includes the runtime environment. Similarly, operating system 1330 can support containers, and applications 1332 can be in the form of containers, which are lightweight, standalone, executable packages of software that include, e.g., code, runtime, system tools, system libraries and settings for an application.

Further, computer 1302 can be enable with a security module, such as a trusted processing module (TPM). For instance with a TPM, boot components hash next in time boot components, and wait for a match of results to secured values, before loading a next boot component. This process can take place at any layer in the code execution stack of computer 1302, e.g., applied at the application execution level or at the operating system (OS) kernel level, thereby enabling security at any level of code execution.

A user can enter commands and information into the computer 1302 through one or more wired/wireless input devices, e.g., a keyboard 1338, a touch screen 1340, and a pointing device, such as a mouse 1342. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 1304 through an input device interface 1344 that can be coupled to the system bus 1308, but can be connected by other interfaces, such as a parallel port, an IEEE 1394 serial port, a game port, a USB port, an IR interface, a BLUETOOTH^{®} interface, etc.

A monitor 1346 or other type of display device can be also connected to the system bus 1308 via an interface, such as a video adapter 1348. In addition to the monitor 1346, a computer typically includes other peripheral output devices (not shown), such as speakers, printers, etc.

The computer 1302 can operate in a networked environment using logical connections via wired or wireless communications to one or more remote computers, such as a remote computer(s) 1350. The remote computer(s) 1350 can be a workstation, a server computer, a router, a personal computer, portable computer, microprocessor-based entertainment appliance, a peer device or other common network node, and typically includes many or all of the elements described relative to the computer 1302, although, for purposes of brevity, only a memory/storage device 1352 is illustrated. The logical connections depicted include wired/wireless connectivity to a local area network (LAN) 1354 or larger networks, e.g., a wide area network (WAN) 1356. Such LAN and WAN networking environments are commonplace in offices and companies, and facilitate enterprise-wide computer networks, such as intranets, all of which can connect to a global communications network, e.g., the Internet.

When used in a LAN networking environment, the computer 1302 can be connected to the local network 1354 through a wired or wireless communication network interface or adapter 1358. The adapter 1358 can facilitate wired or wireless communication to the LAN 1354, which can also include a wireless access point (AP) disposed thereon for communicating with the adapter 1358 in a wireless mode.

When used in a WAN networking environment, the computer 1302 can include a modem 1360 or can be connected to a communications server on the WAN 1356 via other means for establishing communications over the WAN 1356, such as by way of the Internet. The modem 1360, which can be internal or external and a wired or wireless device, can be connected to the system bus 1308 via the input device interface 1344. In a networked environment, program modules depicted relative to the computer 1302 or portions thereof, can be stored in the remote memory/storage device 1352. It will be appreciated that the network connections shown are example and other means of establishing a communications link between the computers can be used.

When used in either a LAN or WAN networking environment, the computer 1302 can access cloud storage systems or other network-based storage systems in addition to, or in place of, external storage devices 1316 as described above, such as but not limited to a network virtual machine providing one or more aspects of storage or processing of information. Generally, a connection between the computer 1302 and a cloud storage system can be established over a LAN 1354 or WAN 1356 e.g., by the adapter 1358 or modem 1360, respectively. Upon connecting the computer 1302 to an associated cloud storage system, the external storage interface 1326 can, with the aid of the adapter 1358 or modem 1360, manage storage provided by the cloud storage system as it would other types of external storage. For instance, the external storage interface 1326 can be configured to provide access to cloud storage sources as if those sources were physically connected to the computer 1302.

The computer 1302 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

Various non-limiting aspects are described in the following examples.

EXAMPLE 1: A system comprising: a mass spectrometer that produces a fragmentation spectrum from a sample; and a processor that executes computer-executable components stored in a non-transitory computer-readable memory, wherein the computer-executable components comprise: an annotation component that generates an annotated portion of the fragmentation spectrum based on previously removed annotated ion peaks of the fragmentation spectrum, wherein the annotated portion of the fragmentation spectrum comprises one or more ion peaks annotated as belonging to first molecular structure; and a looping component that removes the annotated ion peaks from the fragmentation spectrum and re-submits the fragmentation spectrum to the annotation component for one or more additional iterations of annotation.

EXAMPLE 2: The system of any preceding example can be implemented, wherein the looping component further checks if a defined search criteria has been met; and, in response to the defined search criteria being met, ends annotation of the fragmentation spectrum.

EXAMPLE 3: The system of any preceding example can be implemented, wherein the defined search criteria comprise at least one of a defined number of annotation iterations, a defined amount of time, a defined number of annotated ion peaks, a defined percentage of total number of ion peaks being annotated, or a similarity metric based on a comparison between the fragmentation spectrum and a fragmentation spectrum of a known compound.

EXAMPLE 4: The system of any preceding example can be implemented, wherein the generating the annotated portion of the fragmentation spectrum comprises: comparing one or more ion peaks of the fragmentation spectrum to one or more ion peaks of one or more possible match fragmentation patterns stored in a pattern library, wherein the pattern library stores fragmentation patterns of one or more known compounds; selecting a match fragmentation pattern from the one or more possible match fragmentation patterns; and marking the one or more matched ion peaks of the fragmentation spectrum as belonging to a molecular substructure associated with the match fragmentation pattern.

EXAMPLE 5: The system of any preceding example can be implemented, wherein the computer-executable components further comprise: an annotation machine learning model that selects the match fragmentation pattern from the one or more possible match fragmentation patterns; and a training component that trains the annotation machine learning model, wherein the training comprises: generating, using the annotation machine learning model, an annotated fragmentation spectrum of a known sample; comparing the annotated fragmentation spectrum to the fragmentation pattern of the known sample; and updating the annotation machine learning model based on results of the comparing.

EXAMPLE 6: The system of any preceding example can be implemented, wherein the mass spectrometer comprises at least one of a quadrupole mass analyzer, an orbitrap mass analyzer, a time-of-flight mass analyzer, or an asymmetric track lossless mass analyzer.

In various aspects, any combination or combinations of EXAMPLES 1-6 can be implemented.

EXAMPLE 7: A computer-implemented method, comprising: annotating, by a device operatively coupled to a processor, one or more ion peaks of a fragmentation spectrum as belonging to a first molecular substructure; removing, by the device, the annotated ion peaks from the fragmentation spectrum; and annotating, by the device, one or more remaining ion peaks of the fragmentation spectrum as belonging to a second molecular substructure, based on the removed annotated ion peaks.

EXAMPLE 8: The computer-implemented method of any preceding example can be implemented, further comprising: checking, by the device, if a defined search criteria has been met; and, in response to the defined search criteria not being met, re-submitting, by the device, the fragmentation spectrum for one or more additional iterations of annotation.

EXAMPLE 9: The computer-implemented method of any preceding example can be implemented, wherein the defined search criteria comprise at least one of a defined number of annotation iterations, a defined amount of time, a defined number of annotated ion peaks, a defined percentage of total number of ion peaks being annotated, or a similarity metric based on a comparison between the fragmentation spectrum and a fragmentation spectrum of a known compound.

EXAMPLE 10: The computer-implemented method of any preceding example can be implemented, further comprising: producing, by a scientific instrument, the fragmentation spectrum from a sample, wherein the scientific instrument comprises a mass analyzer and a fragmentation technology.

EXAMPLE 11: The computer-implemented method of any preceding example can be implemented, wherein the fragmentation technology comprises at least one of collision-induced dissociation, higher-energy collisional dissociation, electron transfer dissociation, electron capture dissociation or ultraviolet photodissociation.

EXAMPLE 12: The computer-implemented method of any preceding example can be implemented, wherein the annotating comprises: comparing, by the device, one or more ion peaks of the fragmentation spectrum to one or more ion peaks of one or more possible match fragmentation patterns stored in a pattern library, wherein the pattern library stores fragmentation patterns of one or more known compounds; selecting, by the device, a match fragmentation pattern from the one or more possible match fragmentation patterns; and marking, by the device, the one or more matched ion peaks of the fragmentation spectrum as belonging to a molecular substructure associated with the match fragmentation pattern.

EXAMPLE 13: The computer-implemented method of any preceding example can be implemented, wherein the selecting the match fragmentation pattern is based on at least one of a scoring algorithm, one or more defined matching criteria, and one or more historical relationships between molecular compounds.

EXAMPLE 14: The computer-implemented method of any preceding example can be implemented, further comprising, training, by the device, an annotation machine learning model to select the match fragmentation pattern from the one or more possible match fragmentation patterns, wherein the training comprises: generating, by the device, using the annotation machine learning model, an annotated fragmentation spectrum of a known sample; comparing, by the device, the annotated fragmentation spectrum to the fragmentation pattern of the known sample; and updating, by the device, the annotation machine learning model based on results of the comparing.

In various aspects, any combination or combinations of EXAMPLES 7-14 can be implemented.

EXAMPLE 15: A computer program product comprising a non-transitory computer-readable memory having program instructions embodied therewith, the program instructions executable by a processor to cause the processor to: annotate a one or more ion peaks of a fragmentation spectrum as belonging to a first molecular substructure; remove the annotated ion peaks from the fragmentation spectrum; and annotated one or more remaining ion peaks of the fragmentation spectrum as belonging to a second molecular substructure, based on the removed annotated ion peaks.

EXAMPLE 16: The computer program product of any preceding example can be implemented, wherein the program instructions executable by the processor further cause the processor to: check if a defined search criteria has been met; and in response to the defined search criteria not being met, re-submit the fragmentation spectrum for one or more additional iterations of annotation.

EXAMPLE 17: The computer program product of any preceding example can be implemented, wherein the program instructions executable by the processor further cause the processor to: produce, using a scientific instrument, the fragmentation spectrum from a sample, wherein the scientific instrument comprises a mass analyzer and a fragmentation technology.

EXAMPLE 18: The computer program product of any preceding example can be implemented, wherein the annotating comprises: comparing one or more ion peaks of the fragmentation spectrum to one or more ion peaks of one or more possible match fragmentation patterns stored in a pattern library, wherein the pattern library stores fragmentation patterns of one or more known compounds; selecting a match fragmentation pattern from the one or more possible match fragmentation patterns; and marking the one or more matched ion peaks of the fragmentation spectrum as belonging to a molecular substructure associated with the match fragmentation pattern.

EXAMPLE 19: The computer program product of any preceding example can be implemented, wherein the selecting the match fragmentation pattern is based on at least one of a scoring algorithm, one or more defined matching criteria, and one or more historical relationships between molecular compounds.

EXAMPLE 20: The computer program product of any preceding example can be implemented, wherein the program instructions executable by the processor further cause the processor to train an annotation machine learning model to select the match fragmentation pattern from the one or more possible match fragmentation patterns, wherein the training comprises: generating using the annotation machine learning model, an annotated fragmentation spectrum of a known sample; compare the annotated fragmentation spectrum to the fragmentation pattern of the known sample; and updating the annotation machine learning model based on results of the comparing.

In various aspects, any combination or combinations of EXAMPLES 15-20 can be implemented.

In various aspects, any combination or combinations of EXAMPLES 1-20 can be implemented.

## Claims

1. A computer-implemented method, comprising:
annotating, by a device operatively coupled to a processor, one or more ion peaks of a fragmentation spectrum as belonging to a first molecular substructure;
removing, by the device, the annotated ion peaks from the fragmentation spectrum; and
annotating, by the device, one or more remaining ion peaks of the fragmentation spectrum as belonging to a second molecular substructure, based on the removed annotated ion peaks.

2. The computer-implemented method of claim 1, further comprising:
checking, by the device, if a defined search criteria has been met; and,
in response to the defined search criteria not being met, re-submitting, by the device, the fragmentation spectrum for one or more additional iterations of annotation.

3. The computer-implemented method of claim 2, wherein the defined search criteria comprise at least one of a defined number of annotation iterations, a defined amount of time, a defined number of annotated ion peaks, a defined percentage of total number of ion peaks being annotated, or a similarity metric based on a comparison between the fragmentation spectrum and a fragmentation spectrum of a known compound.

4. The computer-implemented method of claim 1, claim 2 or claim 3, wherein the annotating comprises:
comparing, by the device, one or more ion peaks of the fragmentation spectrum to one or more ion peaks of one or more possible match fragmentation patterns stored in a pattern library, wherein the pattern library stores fragmentation patterns of one or more known compounds;
selecting, by the device, a match fragmentation pattern from the one or more possible match fragmentation patterns; and
marking, by the device, the one or more matched ion peaks of the fragmentation spectrum as belonging to a molecular substructure associated with the match fragmentation pattern.

5. The computer-implemented method of claim 4, wherein the selecting the match fragmentation pattern is based on at least one of a scoring algorithm, one or more defined matching criteria, and one or more historical relationships between molecular compounds.

6. The computer-implemented method of claim 4, further comprising, training, by the device, an annotation machine learning model to select the match fragmentation pattern from the one or more possible match fragmentation patterns, wherein the training comprises:
generating, by the device, using the annotation machine learning model, an annotated fragmentation spectrum of a known sample;
comparing, by the device, the annotated fragmentation spectrum to the fragmentation pattern of the known sample; and
updating, by the device, the annotation machine learning model based on results of the comparing.

7. The computer-implemented method of any preceding claim , further comprising:
producing, by a scientific instrument, the fragmentation spectrum from a sample,
wherein the scientific instrument comprises a mass analyzer and a fragmentation technology.

8. The computer-implemented method of claim 7, wherein the fragmentation technology comprises at least one of collision-induced dissociation, higher-energy collisional dissociation, electron transfer dissociation, electron capture dissociation or ultraviolet photodissociation.

9. A computer program comprising program instructions executable by a processor to cause the processor to carry out the method steps of any one of the preceding claims.

10. A computer program product comprising a non transitory computer readable memory within which is embodied the computer program of claim 9

11. A system comprising a mass spectrometer that produces a fragmentation spectrum from a sample, and a processor that executes program instructions that carry out the method steps of any of claims 1-6.

12. The system of claim 11, wherein the mass spectrometer comprises at least one of a quadrupole mass analyzer, an orbitrap ^{™} mass analyzer, a time of flight mass analyzer, or an asymmetric track lossless mass analyzer.

13. The system of claim 11 or claim 12, further comprising a fragmentation device that uses at least one of collision-induced dissociation, higher-energy collisional dissociation, electron transfer dissociation, electron capture dissociation or ultraviolet photodissociation to fragment precursor ions so as to generate the said fragmentation spectrum.
